(19) Europäisches Patentamt
European Patent Office
Office européen des brevets

(11) Publication number: **0 128 670 A1**

# (12) EUROPEAN PATENT APPLICATION

(21) Application number: **84303213.7**

(22) Date of filing: **11.05.84**

(51) Int. Cl.³: **C 07 H 15/24**, A 61 K 31/70

(30) Priority: **13.05.83 US 494210**

(43) Date of publication of application: **19.12.84**
**Bulletin 84/51**

(84) Designated Contracting States: **AT BE CH DE FR GB IT LI LU NL SE**

(71) Applicant: **ADRIA LABORATORIES INC., P.O. Box 16529, Columbus Ohio (US)**

(72) Inventor: **Carter, John Paul, 4724 Braddock Court Apt. No. B, Columbus Ohio 43220 (US)**
Inventor: **Wolgemuth, Richard L., 10202 Kimberly Drive, Plain City Ohio 43064 (US)**
Inventor: **Carrano, Richard Alfred, 16505 Meredith State Road, Sunbury Ohio 43074 (US)**

(74) Representative: **Warren, Keith Stanley et al, BARON & WARREN 18 South End Kensington, London W8 5BU (GB)**

(54) **4-Demethoxy-3'-deamino-3'(4-morpholinyl) derivatives of anthracycline anticancer antibiotics.**

(57) 3'-deamino-3'-(4-morpholinyl) and 3'-deamino-3'-(3-cyano-4-morpholinyl) derivatives of 4-demethoxydaunorubicin and 4-demethoxydoxorubicin together with their 13-dihydro analogues and their pharmaceutically acceptable acid addition salts are disclosed. These compounds have utility as antitumor agents.

EP 0 128 670 A1

0128670

# 4-DEMETHOXY-3'-DEAMINO-3'(4-MORPHOLINYL) DERIVATIVES OF ANTHRACYCLINE ANTICANCER ANTIBIOTICS

The present invention relates to 3'-deamino-3'-(4-morpholinyl) and 3'-deamino-3'-(3-cyano-4-morpholinyl) derivatives of the known anticancer antibiotics 4-demethoxydoxorubicin and 4-demethoxydaunorubicin.

3'-Deamino-3'-(4-morpholinyl) derivatives of daunorubicin and doxorubicin and their 13-dihydro analogues, namely 3'-deamino-3'-(4-morpholinyl)-13-dihydrodaunorubicin and 3'-deamino-3'-(4-morpholinyl)-13-dihydrodoxorubicin, are described by Acton et al in U.S. Patent No. 4,301,277 and have been shown to have activity with respect to Lymphocytic Leukemia P-388.

Umezawa et al, U.S. Patent No. 4,374,980 discloses a synthesis for the 3'morpholino derivatives of these anthracyclines wherein bis(2-iodoethyl)-ether is reacted with daunomycin, adriamycin or carminomycin in a double alkylation procedure.

The compounds of the present invention are believed to provide therapeutic advantages over either the 4-demethoxy or the 3'-deamino-3'-(4-morpholinyl) derivatives of doxorubicin or daunorubicin.

The present invention provides 3'-deamino-4-demethoxy-3'-(4-morpholinyl) derivatives of daunomycin and doxorubicin of the formula (I):

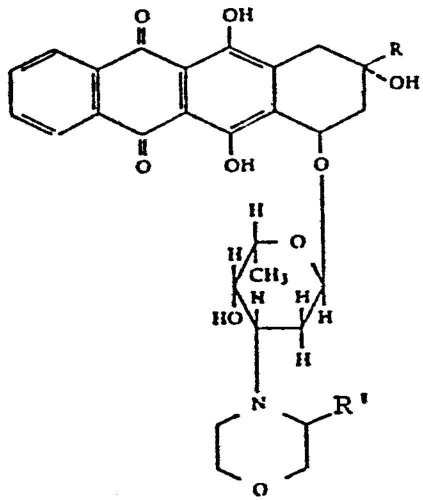

(I)

wherein R is $COCH_3$, $COCH_2OH$, $CH(OH)CH_3$, or $CH(OH)CH_2OH$ and R' is a hydrogen atom or a cyano group. The compounds of the present invention can be prepared in either the free base or the acid addition salt form. Suitable acid addition salts include salts with such acids as hydrochloric, hydrobromic, nitric, sulphuric, phosphoric, glycolic, maleic, hydroxymaleic, malic, tartaric, citric, and methanesulphonic acids and the like.

The present invention also provides therapeutic preparation wherein the aforesaid compounds are used in a non-toxic carrier in therapeutically effective amounts.

Such preparations can be prepared by dispersing or dissolving the compound in any pharmaceutically acceptable non-toxic carrier suitable for the desired mode of administration. Therapeutic compositions of the present invention may be administered parenterally by

intravenous, intraperitoneal, or other conventional injection or orally in some cases. If the carrier is an aqueous medium it may be buffered to pH 7.2-7.5, the physiological range. Any suitable conventional buffer can be used such as tris phosphates, bicarbonates or citrates. If desired, saline solution can be used. A proposed dosage for the compounds is in the range of 1 to 100 $mg/m^2$.

The compounds of the present invention can be prepared by reacting 4-demethoxydaunorubicin and 4-demethoxydoxorubicin in either the N-alkylation synthesis of Umezawa et al or the reductive alkylation of Acton et al as illustrated below. Syntheses for the starting compounds are described in the literature. For example, 4-demethoxydaunorubicin can be prepared by following the teachings of Arcamone et al in U.K. Patent No. 1,509,875 and 4-demethoxydoxorubicin can be prepared by following the teachings in U.K. Patent No. 1,511,680.

In the N-alkylation synthesis, the morpholino ring is formed by reacting the 4-demethoxy derivatives identified above with a bis-(2-haloethyl)ether such as bis-(2-iodoethyl)ether or an ethyl ether having two suitable terminal leaving groups. The 3'-amino group in the compound is sequentially alkylated to yield the morpholinyl ring. The reaction is conveniently carried out at toom temperature in a solvent such as anhydrous dimethylformamide.

Using Acton's synthesis, 4-demethoxy derivatives obtained as above are reacted with 2,2'-oxydiacetaldehyde in the presence of the reducing agent, sodium

cyanoborohydride, under acidic conditions. The reaction proceeds with the formation of a Schiff's base at the 3'-N- position which is reduced in situ to form the secondary amine. Upon further reaction the aldehyde reacts with the secondary amine to form a second Schiff's base which is similarly reduced and the morpholino ring is formed. In some cases, during the reduction of the second Schiff's base, a cyano group is added at the 3-position of the morpholino ring. Additionally, the cyanoborohydride can also reduce the keto-group at C-13. Accordingly, a mixture of compounds is obtained consisting of the 3'-(4-morpholinyl) derivative, the 3'-(3-cyano-4-morpholinyl) derivative, the 3'-(4-morpholinyl)-13-dihydro derivative and the 3'-(3-cyano-4-morpholinyl)-13-dihydro derivative all of which can be separated by HPLC. It will also be noted that the cyanomorpholinyl compound occurs as a mixture of 4 diastereomers of which certain ones may be found to be more active than others.

The preparation of the daunorubicin derivatives of the present invention using both syntheses is illustrated in Examples 1 and 2 below. Example 2 is a proposed synthesis. The doxorubicin derivatives can be obtained in an analogous manner by using 4-demethoxydoxorubicin in place of 4-demethoxydaunorubicin in the procedures disclosed below.

Example 1

3'Deamino-4-Demethoxy-3'-morpholinodaunorubicin

4-Demethoxydaunorubicin hydrochloride (0.04 grams) was dissolved in 1 ml of anhydrous dimethylformamide at room temperature. 0.4 Grams of β,β-diiododiethyl ether was added, followed by 0.03 ml of

triethylamine, and the mixture was stirred overnight. After sixteen hours at room temperature the mixture was poured into 40 ml of water and then extracted three times with methylene chloride. The organic layers were combined, dried over $Na_2SO_4$ and the solvent removed. The residue was chromatographed on silica gel using a 5% solution of methanol in methylene chloride as the eluent. The purified sample was dissolved in ether and a few drops of ethanolic hydrochloric acid was added. The resulting crystals (16 milligrams) were collected and dried under vacuum.

## Example 2

3'Deamino-4-demethoxy-3'-morpholinodaunorubicin, 3'-deamino-4-demethoxy-3'-(3-cyano-4-morpholino) daunorubicin and their 13-dihydro analogues

12 ml of a 4% solution of 2,2'-oxydiacetaldehyde bis(diethyl) acetal is dissolved in 10 ml distilled water containing 0.15 ml of glacial acetic acid. The mixture is gently warmed for 1/2 hour, cooled and the resin filtered off.

10 ml ethylene glycol dimethyl ether is added and the pH of the solution is adjusted to 4 with 0.5 M hydrochloric acid. 534 mg of 4-demethoxydaunorubicin is added followed by the addition of four equivalents of sodium cyanoborohydride. After 3 hours, the pH of the mixture is adjusted to 7 with 5% aqueous sodium bicarbonate. The mixture is extracted 4 times with 10 ml methylene chloride. The combined organic layers are dried (sodium sulfate) and the solvent removed under reduced pressure. The residue is chromatographed on a reverse phase high performance liquid chromatography column packed

with 25 to 40 micrometer C-18 silica gel using a mixture of acetonitrile and water as the eluent. Elution affords the 4-morpholinyl derivative, the 3-cyano-4-morpholinyl derivative, the 4-morpholinyl-13-dihydro derivative and the 3-cyano-4-morpholinyl-13-dihydro derivative.

The principal screen used at the National Cancer Institute for screening compounds against animal tumors is the P388 Lymphocytic Leukemia model. 3'-Deamino-4-demethoxy-3'-(4-morpolinyl)-daunorubicin obtained as above was used in this screen to determine its antitumor activity as follows:

Female CDF1 mice and female DBA2 mice were obtained from Laboratory Animal Supply Company, Indianapolis, Indiana. They were housed in gang stainless steel cages in environmentally controlled animal facilities and fed Purina Laboratory Chow. Water and food were available ad libitum and all mice were allowed at least 1 week for adaptation to their surroundings before they were assigned to a study.

The tumor was maintained by continuous passage in the DBA2 mice. On Day 0, ascitic fluid was removed from a DBA2 mouse, diluted with Hank's balanced salt solution, counted on a Coulter counter (Model MHR) and implanted intraperitoneally (0.2 ml) in CDF2 mice. Twenty-four hours later, mice were randomly distributed into treatment groups and administered drug ip in a total volume of 0.2 ml. Each group of mice was observed daily for 30 days. At a dosage of 0.4 mg/Kg the therapeutic ratio T/C is over 140 as calculated by standard methods.

CLAIMS

1. A compound of the formula (I)

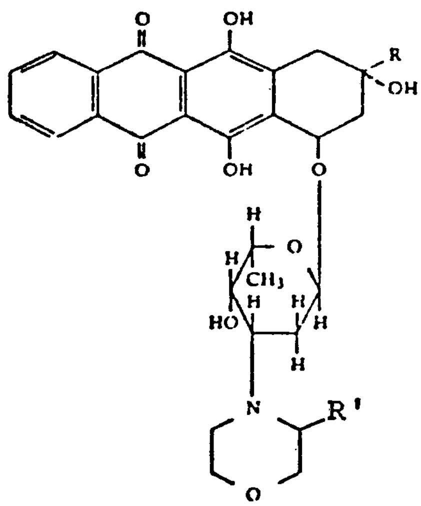

where R is $COCH_3$, $COCH_2OH$, $CH(OH)CH_3$, or $CH(OH)CH_2OH$ and R' is a hydrogen atom or a cyano group and its pharmaceutically acceptable acid addition salts.

2. The compound 3'-deamino-4-demethoxy-3'-(4-morpholinyl)-daunorubicin of claim 1 and its pharmaceutically acceptable acid addition salts.

3. The compound 3'-deamino-4-demethoxy-3'-(3-cyano-4-morpholinyl)-daunorubicin of claim 1 and its pharmaceutically acceptable acid addition salts.

4. The compound 3'-deamino-4-demethoxy-3'-(4-morpholinyl)-13-dihydrodaunorubicin of claim 1 and its pharmaceutically acceptable acid addition salts.

5. The compound 3'-deamino-4-demethoxy-3'-(3-cyano-4-morpholinyl)-13-dihydrodaunorubicin of claim 1 and its pharmaceutically acceptable acid addition salts.

6. The compound 3'-deamino-4-demethoxy-3'-(4-morpholinyl)-doxorubicin of claim 1 and its pharmaceutically acceptable acid addition salts.

7. The compound 3'-deamino-4-demethoxy-3'-(3-cyano-4-morpholinyl)-doxorubicin of claim 1 and its pharmaceutically acceptable acid addition salts.

8. The compound 3'-deamino-4-demethoxy-3'-(4-morpholinyl)-13-dihydrodoxorubicin and its pharmaceutically acceptable acid addition salts.

9. A pharmaceutical preparation comprising, in a non-toxic pharmaceutically acceptable carrier, a therapeutically effective amount of a compound of the formula (I)

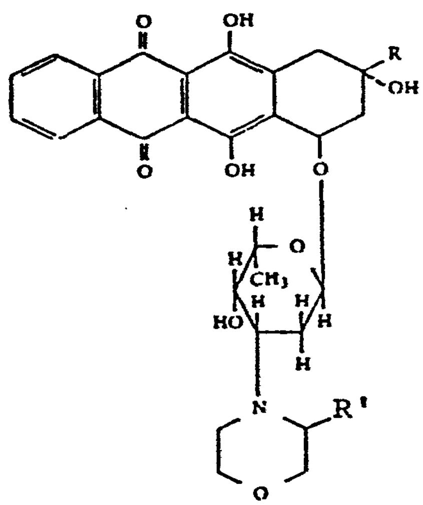

where R is $COCH_3$, $COCH_2OH$, $CH(OH)CH_3$, or $CH(OH)CH_2OH$ and R' is a hydrogen atom or a cyano group or a pharmaceutically acceptable acid salt thereof.

11. The pharmaceutical preparation of claim 10 wherein said compound is 3'-deamino-4-demethoxy-3'-(4-morpholinyl)-daunorubicin or a pharmaceutically acceptable acid addition salt thereof.

12. The pharmaceutical preparation of claim 10 wherein said compound is 3'-deamino-4-demethoxy-3'-(3-cyano-4-morpholinyl)-daunorubicin or a pharmaceutically acceptable acid addition salt thereof.

13. The pharmaceutical preparation of claim 10 wherein said compound is 3'-deamino-4-demethoxy-3'-(4-morpholinyl)-13-dihydrodaunorubicin or a pharmaceutically acceptable acid addition salt thereof.

14. The pharmaceutical preparation of claim 10 wherein said compound is 3'-deamino-4-demethoxy-3'-(3-cyano-4-morpholinyl)-13-dihydrodaunorubicin or a pharmaceutically acceptable acid addition salt thereof.

15. The pharmaceutical preparation of claim 10 wherein said compound is 3'-deamino-4-demethoxy-3'-(4-morpholinyl)-doxorubicin or a pharmaceutically acceptable acid addition salt thereof.

16. The pharmaceutical preparation of claim 10 wherein said compound is 3'-deamino-4-demethoxy-3'-(3-cyano-4-morpholinyl)-doxorubicin or a pharmaceutically acceptable acid addition salt thereof.

17. The pharmaceutical preparation of claim 10 wherein said compound is 3'-deamino-4-demethoxy-3'-(4-morpholinyl)-13-dihydrodoxorubicin or a pharmaceutically acceptable acid addition salt thereof.

18. The pharmaceutical preparation of claim 10 wherein said compound is 3'-deamino-4-demethoxy-3'-(3-cyano-4-morpholinyl)-13-dihydrodoxorubicin or a pharmaceutically acceptable acid addition salt thereof.

19. A process of preparing the compound or pharmaceutical preparation, as the case may be, according to any one of the preceding claims and substantially as hereinbefore described.

European Patent Office

# EUROPEAN SEARCH REPORT

0128670
Application number

EP 84 30 3213

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,Y | US-A-4 301 277 (E.M. ACTON) * Claims 1,2 * --- | 1,9 | C 07 H 15/24<br>A 61 K 31/70 |
| D,Y | US-A-4 374 980 (H. UMEZAWA) * Claims 1,3 * --- | 1,9 | |
| D,Y | GB-A-1 509 875 (SOC. PHARMAC. ITALIA) * Page 3, example 4 * --- | 1,9 | |
| Y | DE-A-2 652 391 (SOC. PHARMAC. ITALIA) * Page 9 * & GB - A - 1 511 680 (Cat. D) --- | 1,9 | |
| P,X | GB-A-2 124 224 (SRI) * Page 18, lines 20-35; page 6, lines 57-58; page 7, line 1; page 13, lines 53-63 * ----- | 1-19 | **TECHNICAL FIELDS SEARCHED (Int. Cl. 3)**<br>C 07 H 15/00<br>A 61 K 31/00 |

The present search report has been drawn up for all claims

| Place of search | Date of completion of the search | Examiner |
|---|---|---|
| THE HAGUE | 29-08-1984 | VERHULST W. |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82